# NOUVEAU FASCICULE DE BREVET EUROPEEN

(11) **EP 0 278 877 B2**
(45) Date de publication et mention de la décision concernant l'opposition: **31.05.2006**
(45) Mention de la délivrance du brevet: 17.11.1994
(21) Numéro de dépôt: 88420027.0
(22) Date de dépôt: 28.01.1988
(51) Int. Cl.: A61K 39/35, A61K 9/20, A61K 9/18

(54) **Formes galéniques d'allergènes pour administration par voie sublinguale**
Galenische Formen von Allergenen für die sublinguale Verabreichung
Galenical forms of allergens for sublingual administration

(30) Priorité: 28.01.1987 FR 8701378
(43) Date de publication de la demande: 17.08.1988
(73) Titulaire: STALLERGENES SA, 92183 Antony Cedex (FR)
(72) Inventeur: Bruttmann, Georges, F-38000 Grenoble (FR); Pedrali, Patrick, F-74000 Annecy le Vieux (FR); Robert, Serge, Braine le Chateau (BE)
(74) Mandataire: Blot, Philippe Robert Emile

(56) Documents cités:
- EP-A- 0 192 321
- G. NETIEN et al., "Galenica 16 - Médicaments homéopathiques", édition 2, 1986, Technique et Documentation, Paris (FR); pp. 77-99#

## Description

La présente invention concerne un médicament comprenant une forme galénique solide d'un allergène pour administration par voie sub-linguale.

On connaît toute l'importance que présentent, depuis une période déjà ancienne, les techniques d'immunothérapie active et spécifique de désensibilisation avec utilisation d'allergènes.

Plusieurs techniques de désensibilisation sont actuellement proposées, la plus courante étant l'application sous forme de solutions injectables. Tous les praticiens connaissent les inconvénients de cette forme d'administration. Il faut, de plus, noter la non-fiabilité des dosages utilisés.

On peut, en effet, observer des variations de concentration importantes dans des flacons renfermant les mêmes concentrations théoriques, et ce, selon les lots de fabrication. Les doses injectées restent donc très approximatives, comme le sont les dilutions indiquées par les fabricants.

On connaît, par ailleurs, la durée des traitements de désensibilisation par voie injectable : il faut, en général, deux interventions par semaine en début de traitement, puis une intervention par semaine, une intervention tous les dix à douze jours, une intervention tous les quinze à vingt jours, le traitement se poursuivant pendant plusieurs années.

Il s'agit donc là de traitements longs et pénalisants pour le patient qui, souvent, les abandonne avant qu'ils arrivent à leur fin.

Une abondante littérature a déjà été consacrée aux traitements de désensibilisation par voie orale. Récemment G. MOLLER et Al dans Allergy 1986, 41, 271-278 a pu déterminer que l'on pouvait obtenir des résultats satisfaisants (avec toutefois des inconvénients liés à des troubles gastro-intestinaux) par administration de capsules à revêtement entérique mais au prix de dosages très élevés d'allergène, atteignant quatre-vingt fois ceux nécessaires dans les traitements par injection. Il semble que, de plus, l'on n'arrive pas à déterminer quelle est la quantité d'antigène absorbée qui est réellement désorbée et il est donc extrêmement difficile d'établir une posologie exacte.

On a, par ailleurs, souligné l'intérêt que peut présenter une application par voie sub-linguale (Glenis K. et al - Clinical Allergy, 1986, Vol.16, 483-491) qui permettrait d'éviter certains effets secondaires des applications par voie injectable.

La forme galénique choisie (forme liquide) ne saurait toutefois donner entière satisfaction ; quand on arrive à des dosages (donc à des volumes) relativement importants, une certaine partie du liquide est absorbée par voie orale et échappe donc à l'application par voie sub-linguale, ce qui fait qu'il n'est pas possible de maîtriser tous les facteurs du traitement.

Il s'agit d'une forme galénique assurant des dosages très approximatifs.

Cette méthode d'application par voie sub-linguale semble pourtant très prometteuse car les IgA salivaires doivent jouer un rôle important dans l'efficacité du traitement ; le mécanisme de la désensibilisation par voie sub-linguale est, de plus, sensiblement le même que celui de la désensibilisation par voie injectable.

Conformément au document suivant : "G.NETIEN et al - Galenica 16 - Médicaments homéopatiques, édition 2, 1986, pages 77-99 - Technique et Documentation, Paris, pages 78, 85, 87, 94 et 98", on a décrit un procédé d'obtention de formes galéniques solides, de diverses substances, dont certaines peuvent être tenues pour des allergènes, ces formes comprenant un support solide pharmaceutiquement acceptable, prévu pour une libération progressive de ladite substance, ainsi que cette dernière sur ledit support.

Les inventeurs on apporté tous leurs efforts à l'étude de nouvelles formes galéniques d'allergènes permettant de tirer partie de tous les avantages du mode d'administration par voie sublinguale sans présenter pour autant les inconvénients mentionnés ci-avant.

Selon la présente invention, on a découvert de nouveaux médicaments de désensibilisation, comprenant un support solide adapté à une administration par voie sublinguale, et une quantité ou dose strictement contrôlée et reproductible d'un allergène, présente sur ledit support.

Cette dose est susceptible d'être obtenue par le procédé comprenant les étapes suivantes :
- mise en solution de l'allergène dans un solvant pour obtenir une solution-mère ;
- préparation à partir de cette solution-mère de plusieurs dilutions à des concentrations respectivement différentes ;
- fractionnement de chaque dilution en plusieurs sous-dilutions, à des concentrations respectivement différentes ;
- imprégnation du support solide avec une dilution ou sous-dilution.

Les médicaments selon l'invention, assurent un dosage progressif et rigoureux de l'allergène, qui permet donc d'assurer une surveillance rigoureuse du traitement ; les inventeurs ont, de plus, pu déterminer, ce qui est tout-à-fait surprenant, que ces nouveaux médicaments pour l'application sublinguale permettaient d'obtenir une désensibilisation beaucoup plus rapide que celle que l'on peut obtenir en utilisant les autres formes galéniques d'administration d'allergènes connus, qu'il s'agisse d'administration par voie orale ou par voie sublinguale avec un support qui est liquide.

Le procédé d'obtention des nouvelles formes galéniques d'allergènes selon l'invention va maintenant être expliqué en détail.

Le produit de départ est un allergène lyophilisé.

Cet allergène peut être de tout type : poussière de maison, acariens, hyménoptères, pollen de graminées ou tout autre, qu'il s'agisse de pneumallergènes, ou bien d'allergènes microbiens ou alimentaires.

L'allergène lyophilisé est mis en solution dant tout solvant convenable, de façon à obtenir une solution mère à 100 IR (indice de réaction). Le solvant utilisé peut être l'eau ou le sérum physiologique ; il peut être également choisi parmi d'autres solvants, de préférence polaires, tels que l'alcool éthylique de titre faible.

On prépare ensuite, en utilisant le même solvant que celui qui a servi à obtenir la solution-mère des dilutions à 10 IR, 1 IR, 0,1 IR, 0,01 IR, 0,001 IR et 0,0001 IR.

Chacune de ces dilutions est ensuite fractionnée en 1/15è ou 1/20è d'IR, de telle sorte qu'une dilution donne elle-même naissance à quinze ou vingt sous-dilutions.

Toutes les précautions seront prises, lors de chacune de ces dilutions ou sous-dilutions, pour que la quantité de solvant porteur d'allergène reste constante, ceci afin de réaliser toutes les imprégnations de façon identique.

On réalise ensuite, dans une turbine, à l'aide d'un injecteur du type dénommé "spray-doseur", l'imprégnation, à l'aide de chacune des dilutions ou sous-dilutions de globules constitués, de façon connue en soi, d'un support solide pharmaceutiquement acceptable et spécialement d'un mélange saccharose-lactose. Il est bien évident que, sans sortir du cadre de l'invention, ces globules pourraient être remplacés par des poudres ou des comprimés, également adaptés à l'application par voie sublinguale.

La technique d'imprégnation est celle de la multi-imprégnation ou de l'imprégnation fractionnée, de manière à garantir une parfaite répartition du principe actif homogène.

Entre chaque imprégnation, le séchage est réalisé par passage d'air forcé et desséché dans une chambre où règne une dépression obtenue par la différence de débit existant entre ce passage d'air et un puissant extracteur.

Selon une caractéristique importante de l'invention, cette opération de séchage sera effectuée à une température inférieure ou égale à 30 °C.

On a, en effet, déterminé, au cours des nombreux essais ayant conduit à la mise au point dudit procédé, que le procédé n'est plus fiable quand la température dépasse 38-40 ° C.

La dernière imprégnation est une imprégnation protectrice du type "dragéification".

Selon un autre mode de réalisation du procédé selon l'invention, et afin de garantir que soit totalement maintenue l'intégralité physicochimique du principe actif, les différentes étapes de l'imprégnation sont effectuées sous atmosphère d'azote.

La dernière opération consiste à mettre en gélules les globules ainsi obtenus, ou éventuellement à placer lesdits globules ou les poudres ou comprimés dans des tubes-doses et à terminer le conditionnement de façon classique (blisters pour les gélules, coffrets pour les tubes-doses) en numérotant, bien évidemment, les gélules à doses croissantes.

Chaque fraction IR étant divisée en 1/20è, les préparations terminales se présentent sous forme de plaquettes-calendriers à dose croissante de quarante ou cinquante jours de traitement.

Il suffit au patient de prendre une prise quotidienne en doses croissantes pendant quatre-vingt à cent jours pour arriver à la dose maximale.

Il prend ensuite des doses dites de stabilisation avec une dose par jour, puis deux fois par semaine, puis une fois par semaine.

On a constaté que, de façon très suprenante, par rapport à la désensibilisation classique et notamment par rapport à la désensibilisation par injection, les résultats cliniques sont très fortement supérieurs ; on relève une amélioration de 80 à 85 % au bout de deux à trois mois de traitement. La production d'anticorps bloquants du type IgG4 est accélérée et la tolérance est parfaite.

On n'a dénombré que 2 à 3 % de réactions syndromiques ; celles-ci sont facilement contrôlées par une diminution -ou un espacementdes prises.

Un autre avantage tout à fait surprenant des nouvelles formes galéniques d'allergènes selon l'invention est que la durée de la désensibilisation est très fortement réduite par rapport aux autres formes galéniques d'application connues. Le traitement ne dure plus plusieurs années mais simplement quelques mois, et surtout, il est possible d'atteindre les doses maximales très rapidement, entre quatre-vingt et cent jours.

Les nouvelles formes galéniques d'allergènes selon l'invention présentent encore d'autres avantages parmi lesquels on peut citer l'allègement important du coût de la désensibilisation, compte-tenu de la quasi-disparition des actes médicaux ou des soins infirmiers. Seules sont nécessaires des consultations de contrôle à un mois ou deux mois.

Des essais, menés en double aveugle contre placebo sur plus de deux mille patients, ont permis de confirmer ce qui précède.

Certains de ces résultats sont rassemblés dans les tableaux qui suivent :

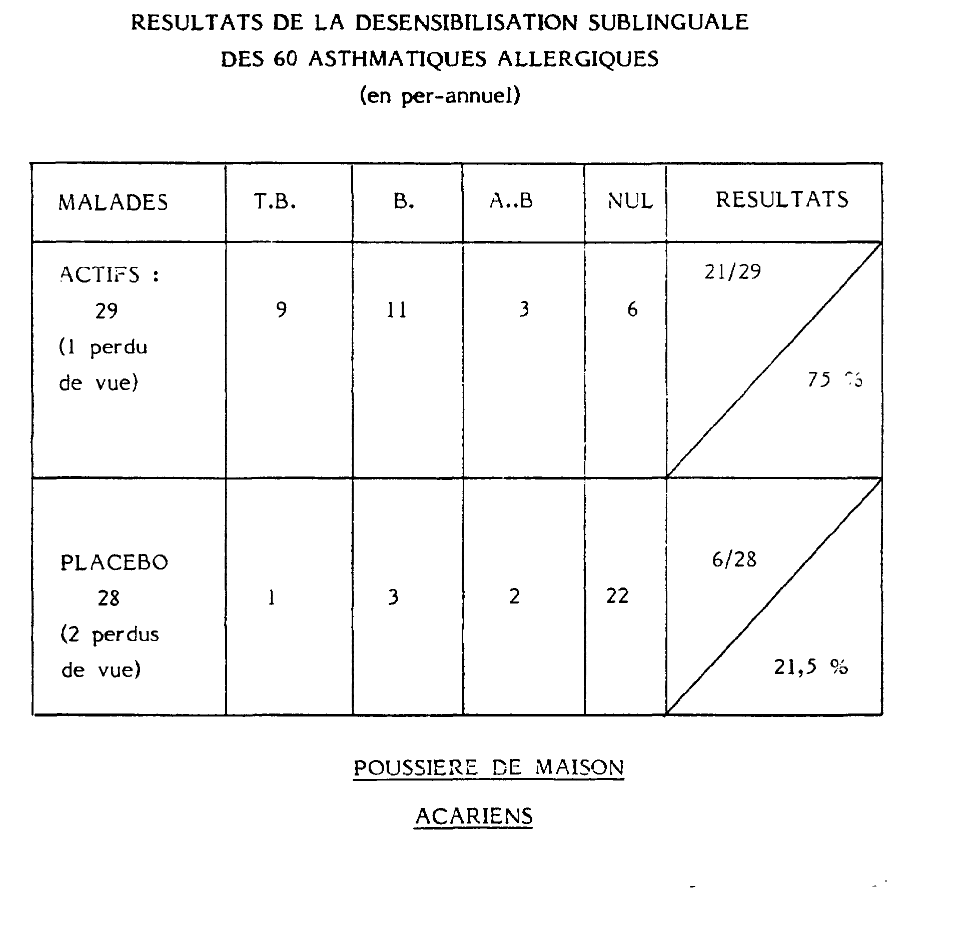

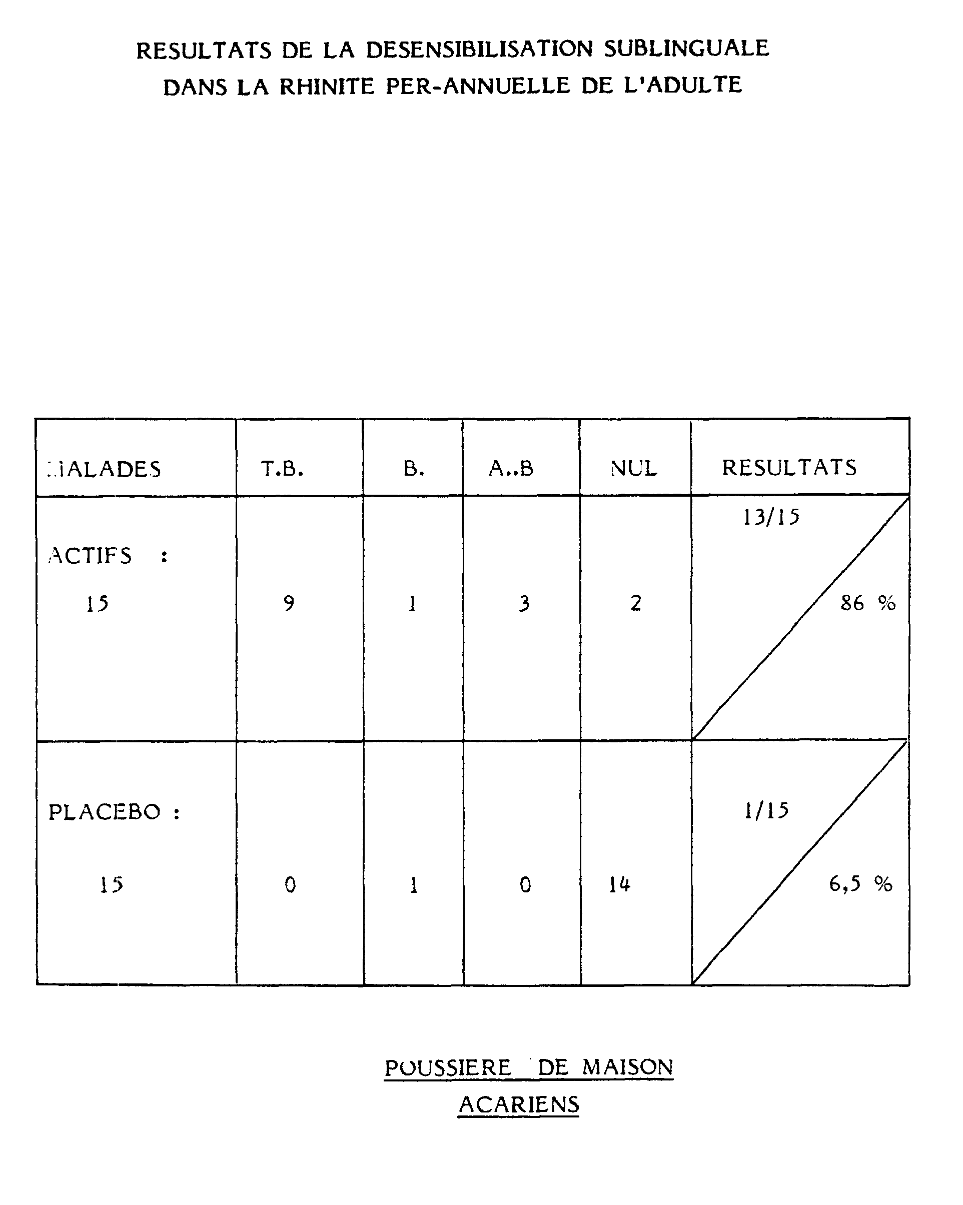

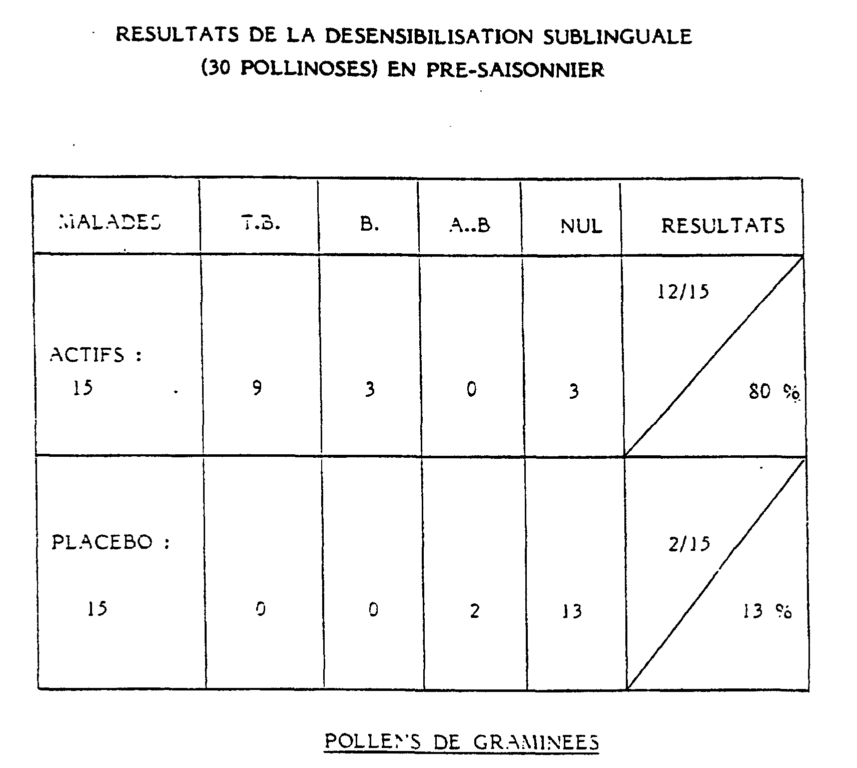

Il est bien évident que la présente invention ne saurait se limiter aux concentrations, modes de fractionnement ou dilutions donnés ci-avant à titre d'exemple non limitatif ; ces concentrations, modes de fractionnement et dilutions peuvent, bien entendu, être adaptés sur demande ; de même, le support saccharose-lactose peut être remplacé par un autre support pharmaceutiquement acceptable adapté.

## Revendications

1. Utilisation d'un allergène pour la préparation d'un médicament de désensibilisation progressive par voie d'administration sublinguale, **caractérisée en ce qu'**une quantité ou dose strictement contrôlée dudit allergène est présente dans un support solide pharmaceutiquement acceptable et adapté à une administration par voie sublinguale.

2. Utilisation selon la revendication 1, **caractérisée en ce que** le médicament comprend plusieurs doses de l'allergène, conditionnées sur une même unité de conditionnement, telle qu'une plaquette, sur laquelle sont numérotés les dosages croissants.

3. Utilisation selon la revendication 1, **caractérisée en ce que** le produit de départ est un allergène lyophilisé.

4. Utilisation selon la revendication 1, **caractérisée en ce que** le support solide est un mélange saccharose-lactose.

## Patentansprüche

1. Verwendung eines Allergens zur Herstellung eines Arzneimittels für die progressive Desensibilisierung auf dem Weg der sublingualen Verabreichung, **dadurch gekennzeichnet, dass** eine genau kontrollierte Menge oder Dosis des Allergens in einem pharmazeutisch annehmbaren festen Träger vorliegt und für eine Verabreichung auf sublingualem Weg ausgelegt ist.

2. Verwendung nach Anspruch 1, **dadurch gekennzeichnet, dass** das Arzneimittel eine Vielzahl von Dosen des Allergens aufweist, die auf ein und derselben Konditioniereinheit, wie einer Platte, konditioniert sind, auf welcher die stärker werdenden Dosen nummeriert sind.

3. Verwendung nach Anspruch 1, **dadurch gekennzeichnet, dass** das Ausgangsprodukt ein lyophilisiertes Allergen ist.

4. Verwendung nach Anspruch 1, **dadurch gekennzeichnet, dass** der feste Träger eine Saccharose-Lactose-Mischung ist.

## Claims

1. Use of an allergen for the preparation of a medicinal product for progressive desensitisation by sublingual administration, **characterised in that** a strictly controlled quantity or dose of the said allergen is present in a pharmaceutically acceptable solid vehicle adapted to sublingual administration.

2. Use according to claim 1, **characterised in that** the medicinal product comprises several doses of the allergen, packaged in the same packaging unit, such as a blister pack, on which the increasing dosages are numbered.

3. Use according to claim 1, **characterised in that** the starting product is a lyophilised allergen.

4. Use according to claim 1, **characterised in that** the solid vehicle is a sucrose/lactose mixture.
